# EUROPÄISCHE PATENTSCHRIFT

(11) **EP 0 333 658 B1**
(45) Veröffentlichungstag und Bekanntmachung des Hinweises auf die Patenterteilung: **26.07.1995**
(21) Anmeldenummer: 89810184.5
(22) Anmeldetag: 09.03.1989
(51) Int. Cl.: C07D 317/46, C07D 405/04, C07F 5/02, C07F 7/08

(54) **Verfahren zur Herstellung von substituierten Difuorbenzo-1,3-dioxolen**
Process for the preparation of substituted difluorbenzo-1,3-dioxoles
Procédé pour la préparation de difluorbenzo-1,3-dioxoles substitués

(30) Priorität: 18.03.1988 CH 1044/88
(43) Veröffentlichungstag der Anmeldung: 20.09.1989
(73) Patentinhaber: CIBA-GEIGY AG, 4002 Basel (CH)
(72) Erfinder: Ackermann, Peter, Dr., CH-4148 Pfeffingen (CH); Känel, Hans-Ruedi, Dr., CH-4416 Bubendorf (CH); Schaub, Bruno, Dr., CH-2822 Courroux (CH)

(56) Entgegenhaltungen:
- EP-A- 0 082 681
- EP-A- 0 198 797
- EP-A- 0 206 999
- EP-A- 0 206 999
- EP-A- 0 291 799
- JOURNAL OF ORGANOMETALLIC CHEMISTRY Band 136, Nr. 2, 1977, Seiten 139-146, Lausanne, CH

## Beschreibung

Die Erfindung betrifft ein Verfahren zur Herstellung von in 4-Stellung substituierten 2,2-Difluorbenzo-1,3-dioxolen durch die Umsetzung von 2,2-Difluorbenzo-1,3-dioxol mit einer Alkalimetallverbindung und die anschliessende Umsetzung mit einer elektrophilen Verbindung.

In der EP-A-0 206 999 sind gegebenenfalls N-substituierte 3-(2,2-Difluorbenzo-1,3-dioxol-4-yl)-4-cyanopyrrole beschrieben, die wertvolle Mittel zur Bekämpfung von Mikroorganismen darstellen. Die Herstellung der Verbindungen erfolgt über eine mehrstufige Synthese. Das als Ausgangsprodukt benötigte 3-(2,2-Difluorbenzo-1,3-dioxol-4-yl)-2-propensäurenitril wird durch die Diazotierung von 4-Amino-2,2-difluorobenzo-1,3-dioxol, anschliessende Umsetzung mit Acrylnitril in Gegenwart von CuCl und Dehalogenierung des gebildeten 4-(2-Chlor-2-cyano-eth-1-yl)-2,2-difluorbenzo-1,3-dioxols erhalten.
In der DE-OS 2 819 788 sind z.B. (2,2-Difluorbenzo-1,3-dioxol-4-yl)-methylester von Pyrethroid- oder pyrethroid-ähnlichen Carbonsäuren als Insektizide beschrieben. Die Benzodioxolylmethylalkohole werden hierbei nach üblichen Verfahren, hergestellt, z.B. durch Reduktion von Aldehyden. 2,2-Difluorbenzo-1,3-dioxol-4-carbaldehyd ist in der nachveröffentlichten EP-A-291 799 beschrieben; die hierin offenbarte Synthese ist als technische Herstellungsmethode jedoch ungeeignet.

E.L. Stogryn beschreibt im J. Org. Chem., Vol. 37, S. 673 (1972) die Herstellung von 2,2-Difluorbenzo-1,3-dioxol-5-carbaldehyd durch die Formylierung von 2,2-Difluorbenzo-1,3-dioxol-5-yllithium mit Dimethylformamid. Die Lithiumverbindung erhält man durch die Umsetzung von 5-Brom-2,2-difluorbenzo-1,3-dioxol mit Butyllithium.

S. Cabiddu et al. beschreiben in J. Organometallic Chem., Vol. 136, S. 139 (1977) die Umsetzung von 1,3-Benzodioxol mit n-Butyllithium und anschliessende Umsetzung mit CO₂ zur entsprechenden Benzodioxol-4-yl-carbonsäure.

Eine allgemeine Methode zur Herstellung von 2,2-Difluorbenzo-1,3-dioxolen, die in 4-Stellung unterschiedliche funktionelle Gruppen tragen, ist bisher nicht bekannt geworden.

Es wurde nun gefunden, dass am Benzolkern halogenfreies 2,2-Difluorbenzo-1,3-dioxol mit Alkalimetallverbindungen direkt und mit hoher Stellungsspezifität zu in 4-Stellung metallisiertem 2,2-Difluorbenzo-1,3-dioxol reagiert, das mit elektrophilen Verbindungen weiter zu entsprechenden in 4-Stellung substituierten 2,2-Difluorbenzo-1,3-dioxolen umgesetzt werden kann.

Gegenstand vorliegender Erfindung ist ein Verfahren zur Herstellung von Verbindungen der Formel I 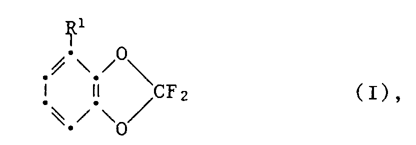
worin R¹ -OH, -SH, -CHO, -CN, -COOH, -B(OH)₂, -COX mit X gleich Cl oder Br, ferner -COOR², -SiR²₃ oder -B(OR²)₂ mit R² in der Bedeutung eines um die Hydroxylgruppe verminderten C₁-C₁₂-Alkoholrestes darstellt, worin R₁ ferner -CₙH₂ₙCOOR² mit n in der Bedeutung einer Zahl von 1 bis 4, oder lineares oder verzweigtes, unsubstituiertes oder mit -F, -CN, C₁-C₆-Alkoxy, Phenyl, Fluorphenyl, C₁-C₄-Alkoxy-phenyl, C₁-C₄-Alkylthio-phenyl, C₁-C₄-Alkyl-phenyl, C₁-C₄-Fluoralkyl-phenyl, Nitrophenyl, Cyanophenyl substituiertes C₁-C₁₂-Hydroxyalkyl bedeutet, oder worin R₁ einen unsubstituierten oder mit F, C₁-C₄-Alkoxy-, C₁-C₄-Alkylthio-, C₁-C₄-Alkyl-, C₁-C₄-Fluoralkyl-, Nitro, oder Cyano substituierten Benzylalkohol oder C₁-C₁₂-Acylrest darstellt, oder worin R₁ ein Rest der Formel II ist, 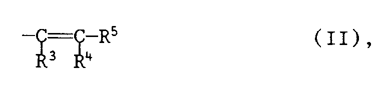
in welchem R⁵ für -CN, -CF₃, -COOR², -CONH₂, -CO-NHR² oder -CONR²₂ steht, R³ und R⁴ eine direkte Bindung oder je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 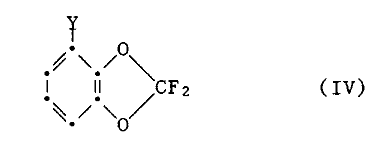 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄, BZ₃ oder ZrZ₄ zu einer Verbindung der Formel IV umsetzt, worin Y -Cu, -MgZ, -ZnZ, -CdZ, -CuZ, -PdZ, -NiZ, -AlZ₂, -SiZ₃, -SnZ, -SnZ₃, -TiZ₃, -BZ₂ oder -ZrZ₃ ist und Z -Cl, -Br oder -I und im Falle von Bor auch -OC₁-₄-Alkyl oder -O-Aryl bedeutet,
b) die Verbindung der Formel IV mit einer elektrophilen Verbindung aus der Gruppe X-CN, CO₂, S₈, COX₂, B(OR²)₃, X-SiR²₃, R²O-SiR²₃ oder XCOOR² mit X gleich -Cl oder -Br, einem Formylierungsreagens, CH₂O, einem Epoxid, einem unsubstituierten oder mit F, C₁-C₄-Alkoxy-, -Alkylthio, -Alkyl oder -Fluoralkyl, Nitro, Cyano substituierten Benzaldehyd, einem unsubstituierten oder mit -F, -Cl, -CN, C₁-C₆-Alkoxy, Phenyl, Fluorphenyl, C₁-C₄-Alkoxyphenyl, C₁-C₄-Alkylthiophenyl, C₁-C₄-Alkylphenyl, C₁-C₄-Fluoralkylphenyl, Nitrophenyl, Cyanophenyl substituierten C₁-C₁₁-Alkyl-CHO oder auch mit C₁-C₁₂-Acyl-X¹, worin X¹ -Cl, -Br, C₁-C₆-Alkoxy oder der Rest eines sekundären Amins ist, X²CₙH₂ₙCOOR² oder X²-CR³=CR⁴R⁵ umsetzt, worin X² eine Abgangsgruppe darstellt, und
c) die Verbindung der Formel I isoliert oder das Reaktionsgemisch zuerst hydrolisiert und dann die Verbindung der Formel I isoliert.

Als bevorzugt gilt dabei ein Verfahren zur Herstellung der Verbindungen der Formel I 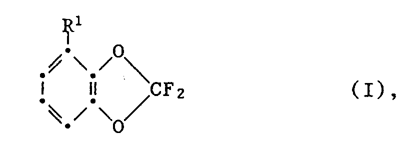
worin R¹ -CHO, -CN, -COOH, -COX mit X gleich Cl oder Br, -COOR² mit R² in der Bedeutung eines um die Hydroxylgruppe verminderten C₁-C₁₂-Alkoholrestes, -CₙH₂ₙCOOR² mit n in der Bedeutung einer Zahl von 1 bis 4, lineares oder verzweigtes, unsubstituiertes oder mit -F, -CN, C₁-C₆-Alkoxy, Phenyl, Fluorphenyl, C₁-C₄-Alkoxyphenyl, C₁-C₄-Alkylthiophenyl, C₁-C₄-Alkylphenyl, C₁-C₄-Fluoralkylphenyl, Nitrophenyl, Cyanophenyl substituiertes C₁-C₁₂-Hydroxyalkyl oder C₁-C₁₂-Acyl, oder ein Rest der Formel II ist, 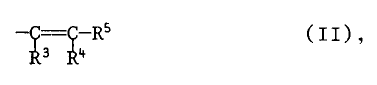
worin R⁵ für -CN, -CF₃, -COOR² oder -CONR²₂ steht, R³ und R⁴ je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 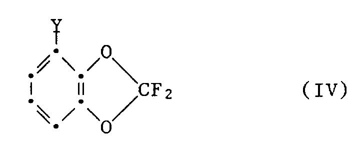 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄ oder ZrZ₄ zu einer Verbindung der Formel IV umsetzt, worin Y -Cu, -MgZ, -ZnZ, -CdZ, -CuZ, -PdZ, -NiZ, -AlZ₂, -SiZ₃, -SnZ, -SnZ₃, -TiZ₃ oder -ZrZ₃ ist und Z -Cl, -Br oder -I bedeutet,
b) die Verbindung der Formel IV mit einer elektrophilen Verbindung aus der Gruppe X-CN, CO₂, COX₂ oder XCOOR² mit X gleich -Cl oder -Br, einem Formylierungsreagens, CH₂O, einem unsubstituierten oder mit -F, -CN, C₁-C₆-Alkoxy, Phenyl, Fluorphenyl, C₁-C₄-Alkoxyphenyl, C₁-C₄-Alkylthiophenyl, C₁-C₄-Alkylphenyl, C₁-C₄-Fluoralkylphenyl, Nitrophenyl, Cyanophenyl substituierten C₁-C₁₁-Alkyl-CHO, einem Epoxid, einem unsubstituierten oder mit -F, C₁-C₄-Alkoxy-, -Alkylthio, -Alkyl oder -Fluoralkyl, Nitro, Cyano substituierten Benzaldehyd, C₁-C₁₂-Acyl-X¹, worin X¹ -Cl, -Br, C₁-C₆-Alkoxy oder der Rest eines sekundären Amins ist, X²CₙH₂ₙCOOR² oder X²-CR³=CR⁴R⁵ umsetzt, worin X² eine Abgangsgruppe darstellt, und
c) die Verbindung der Formel I isoliert oder das Reaktionsgemisch zuerst hydrolisiert und dann die Verbindung der Formel I isoliert.

Ebenfalls bevorzugt ist ein Verfahren zur Herstellung der Verbindungen der Formel I, worin R¹ -CHO, -CN, -COOH, -COX mit X gleich Cl oder Br, oder ein Rest der Formel II ist, 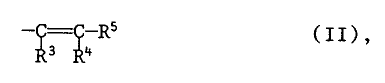
worin R⁵ für -CN, -CF₃, -COOR² oder -CONH₂ steht, R³ und R⁴ je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 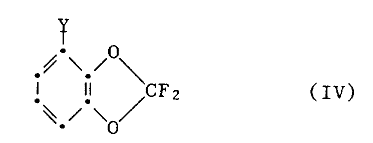 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄, BZ₃ oder ZrZ₄ zu einer Verbindung der Formel IV umsetzt, worin Y -Cu, -MgZ, -ZnZ, -CdZ, -CuZ, -PdZ, -NiZ, -AlZ₂, -SiZ₃, -SnZ, -SnZ₃, -TiZ₃, -BZ₂ oder -ZrZ₃ ist und Z -Cl, -Br oder -I bedeutet,
b) die Verbindung der Formel IV mit einer elektrophilen Verbindung aus der Gruppe X-CN, CO₂ oder COX₂ mit X gleich -Cl oder -Br, einem Formulierungsreagens, oder X²-CR³=CR⁴R⁵ umsetzt, worin X² eine Abgangsgruppe darstellt, und
c) die Verbindung der Formel I isoliert oder das Reaktionsgemisch zuerst hydrolisiert und dann die Verbindung der Formel I isoliert.

Hervorzuheben ist dabei jene Variante zur Herstellung der Verbindungen der Formel I, worin R¹ -CHO, oder ein Rest der Formel II ist, 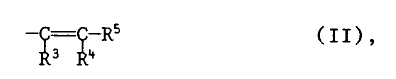
worin R⁵ für -CN, -COOR² oder -CONH₂ steht, R³ und R⁴ je H darstellen, oder R³ H ist und R⁴ unabhängig die Bedeutung von R⁵ hat.

Bei R² in der Bedeutung eines um eine Hydroxylgruppe verminderten Alkoholestes kann es sich z.B. um lineares oder verzweigtes Alkyl mit bevorzugt 1 bis 12, besonders 1 bis 6 C-Atomen, oder um unsubstituiertes, oder mit C₁-C₄-Alkyl substituiertes C₅- oder C₆-Cycloalkyl oder C₅- oder C₆-Cycloalkyl-CₘH₂ₘ- oder Phenyl-CₘH₂ₘ handeln, worin m 0 oder eine Zahl von 1 bis 4, besonders 0, 1 oder 2 ist. Beispiele sind Methyl, Ethyl, die Isomeren von Propyl, Butyl, Pentyl, Hexyl, Octyl, Decyl, Dodecyl, Cyclopentyl, Cyclohexyl, Methylcyclopentyl oder -hexyl, (Methyl- oder Ethylcyclohexyl)methyl, Benzyl, Methyl- oder Ethylbenzyl, Phenylethyl, (Methylphenyl)ethyl.

Bei R¹ in der Bedeutung von CₙH₂ₙCOOR² ist n eine Zahl von 1 bis 4, besonders 1 oder 2. Die -CₙH₂ₙ-Gruppe kann linear oder verzweigt sein. Beispiele für diese Gruppe sind Methylen, Ethylen, Ethyliden, Propyliden, 1,2- oder 1,3-Propylen, Butyliden, 1,2-, 1,3- oder 1,4-Butylen.

R¹ in der Bedeutung von gegebenenfalls substituiertem Hydroxyalkyl enthält bevorzugt 1 bis 6, besonders 1 bis 4 C-Atome. Die Hydroxylgruppe kann an ein primäres, sekundäres oder tertiäres C-Atom gebunden sein. Beispiele für Hydroxyalkyl sind Hydroxymethyl, 1- oder 2-Hydroxyethyl, 1-, 2- oder 3-Hydroxyprop-1- oder -2-yl, 1-, 2-, 3- oder 4-Hydroxybut-1-oder -2-yl, Hydroxypentyl, Hydroxyhexyl, Phenyl(hydroxymethyl), 1-Phenyl-2-hydroxyethyl, Fluorphenyl(hydroxymethyl), 1-Methoxy-2-hydroxyeth-2-yl, 1,1,1-Trifluor-2-hydroxyethyl und Cyanophenyl(hydroxymethyl).

R¹ in der Bedeutung von gegebenenfalls substituiertem Acyl enthält bevorzugt 1 bis 6 und besonders 1 bis 4 C-Atome. Bei dem Acylrest kann es sich z.B. um einen solchen der Formel R⁷-CO- handeln, worin R⁷ unsubstituiertes oder mit -F, -Cl, -CN, C₁-C₆-Alkoxy, Phenyl, Fluorphenyl, C₁-C₄-Alkoxyphenyl, C₁-C₄-Alkylphenyl, C₁-C₄-Alkylthiophenyl, C₁-C₄-Fluoralkylphenyl, Nitrophenyl oder Cyanophenyl substituiertes C₁-C₁₁-, besonders C₁-C₆-Alkyl oder um unsubstituiertes oder mit -F, Cyano, Nitro, C₁-C₄-Alkyl oder -Alkoxy oder -Alkylthio oder -Fluoralkyl substituiertes Phenyl ist. Beispiele sind Formyl, Acetyl, Fluoracetyl, Trifluoracetyl, Chlordifluoracetyl, Propionyl, Butyryl, Benzoyl, Phenylacetyl, 3-Phenylpropionyl oder Fluorbenzoyl.

X¹ enthält als Rest eines sekundären Amins bevorzugt 2 bis 12, besonders 2 bis 8 C-Atome. Der Rest kann der Formel R⁸R⁹N- entsprechen, worin R⁸ C₁-C₆-Alkyl oder -Alkoxy und R⁹ C₁-C₆-Alkyl sind, oder R⁸ C₁-C₄-Alkoxy-C₁-C₃-Alkyl ist und R⁹ C₁-C₆-Alkyl ist oder die Bedeutung von R⁸ hat, oder R⁸ und R⁹ zusammen gegebenenfalls durch -O- oder eine =N-C₁-C₄-Alkylgruppe unterbrochenes Tetra- oder Pentamethylen bedeuten. In einer bevorzugten Ausführungsform handelt es sich bei X¹ um 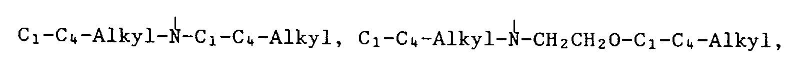
(C₁-C₄-AlkylOCH₂CH₂)₂N- oder den Rest eines 5- oder 6-gliedrigen heterocyclischen Amins, das ein weiteres Heteroatom aus der Gruppe -O-und =N-C₁-C₄-Alkyl enthalten kann. Beispiele sind Dimethylamino, Diethylamino, Methoxymethylamino, Ethoxymethylamino, Methyl(methoxyethyl)amino, Di(methoxymethyl)amino, Pyrrolidino, Piperidino, Morpholino und N-Methylpiperazino.
R⁵ ist vorzugsweise -CN, -CF₃, -COOR² oder -CONH₂.

Bei der Abgangsgruppe X² handelt es sich vorzugsweise um ein Halogenid, besonders -Cl oder -Br, C₁-C₆-Alkoxy, z.B. Methoxy, Ethoxy oder Propoxy, C₆-C₁₀-Aryloxy, z.B. Phenoxy oder Naphthoxy, C₁-C₁₂-, besonders C₁-C₆-Sekundäramino, z.B. Dimethylamino, Diethylamino, Piperidino, C₁-C₈-, besonders C₁-C₄-Acyloxy, z.B. Acetyloxy, oder den Rest einer Sulfonsäure.

Es kann eine aliphatische Sulfonsäure z.B. Methyl-, Ethyl- oder Propylsulfonsäure, oder eine aromatische Sulfonsäure, z.B. Benzol- oder p-Toluolsulfonsäure sein.

2,2-Difluorbenzo-1,3-dioxol ist bekannt und kann z.B. durch Fluorierung von 2,2-Dichlorbenzo-1,3-dioxol erhalten werden.

Die Reaktionsstufe a) wird in einem inerten Lösungsmittel durchgeführt, z.B. in einem unpolaren oder polaren aprotischen Lösungmittel. Geeignete Lösungsmittel sind aliphatische oder aromatische Kohlenwasserstoffe, Ether, tertiäre Amine, N-alkylierte Säureamide, Lactame oder cyclische Harnstoffe, Sulfoxide, Sulfone, Nitrile oder Mischungen hiervon. Beispiele sind Pentan, Isopentan, Hexan, Heptan, Cyclohexan, Methylcyclohexan, Benzol, Toluol, Xylol, Diethylether, Dibutylether, Tetrahydrofuran, Dioxan, Ethylenglykoldimethylether, Triethylamin, N,N,N',N'-Tetramethylethylendiamin, Hexamethylphosphorsäuretriamid, Dimethylformamid, Dimethylacetamid, N-Methylpyrrolidon, Ethylendimethylharnstoff, Propylendimethylharnstoff, Dimethylsulfoxid, Tetramethylensulfon, Acetonitril. Lösungsmittel, die gleichzeitig gemäss Verfahrensstufe b) ein Reaktand sein können, wie z.B. Carbonsäureamide, werden erst in der Verfahrensstufe b) in Form eines Ueberschuss dieser Reaktanden zugegeben.

Die Reaktionstemperatur kann in beiden Verfahrensstufen bevorzugt -150 bis 150°C, besonders -100 bis 80°C betragen.

Bei Verwendung unpolarer oder schwach polarer Lösungsmittel ist es vorteilhaft, dass das Reaktionsgemisch zusätzlich ein Komplexierungsmittel enthält, z.B. in einer Menge von 0,01 Mol-% bis zu einem 10-fachen Ueberschuss, bezogen auf die Verbindung der Formel IV. Solche Komplexierungsmittel können z.B. teritäre Amine oder N-substituierte Säureamide, Lactame oder cyclische Harnstoffe, Ether oder Sulfoxide sein, wie sie zuvor unter den Lösungsmitteln genannt wurden. Im weiteren sind auch Kronenether, wie z.B. 15-Krone-5, 18-Krone-6, Dibenzo-18-Krone-6, Dicyclohexyl-18-Krone-6, Dibenzo-24-Krone-8 oder Dicyclohexyl-24-Krone-8 geeignet. Ferner sind auch Alkalimetall-, besonders Lithium-, Natrium- und Kaliumhalogenide, Magnesiumhalogenid oder Kupferhalogenid geeignet. Halogenid ist bevorzugt Chlorid, Bromid und Jodid.

Bei den stark basischen Anionen kann es sich z.B. um lineares oder verzweigtes C₁-C₆-, besonders C₁-C₄-Alkyl^{⊖}, C₆-C₁₄-Aryl^{⊖}, H₂N^{⊖}, H^{⊖}, C₁-C₆-, besonders C₁-C₄-Alkoxy^{⊖}, C₁-C₆-, besonders C₁-C₄-Alkyl-NH^{⊖} oder Di(C₁-C₆-Alkyl)N^{⊖}, besonders Di(C₁-C₄-Alkyl)N^{⊖} oder Di(C₃-C₆-Cycloalkyl)N^{⊖} handeln. In einer bevorzugten Ausführungsform ist die Alkalimetallverbindung ein Li-, Na-, K-, -Alkyl, -Arylhydrid, -amid oder -alkoholat.

Die Metallierung des 2,2-Difluorbenzo-1,3-dioxols erfolgt in an sich bekannter Weise durch dessen Umsetzung mit einer Alkalimetallverbindung in einem inerten Lösungsmittel bei vorzugsweise 20 bis -80°C. Hierbei kann die Alkalimetallverbindung vorgelegt und das Benzodioxol langsam zugegeben werden. Ebenso kann man umgekehrt verfahren.

Nach dem Reaktionsschritt a) kann direkt die Reaktion b) durchgeführt werden. Man kann aber auch zuvor die Alkalimetallverbindung der Formel IV mit einem wasserfreien Metallhalogenid, z.B. MgZ₂, ZnZ₂, CdZ₂, CuZ, CuZ₂, PdZ₂, NiZ₂, AlZ₃, SiZ₄, SnZ₂, SnZ₄, TiZ₄, BZ₃ oder ZrZ₄, worin Z für Cl, Br oder I, besonders Cl oder Br steht, zu einer Verbindung der Formel IV umsetzen, worin Y für -MgZ, -ZnZ, -CdZ, -CuZ, -PdZ, -NiZ, -AlZ₂, -SiZ₃, -SnZ, -SnZ₃, -TiZ₃, -BZ₂ oder -ZrZ₃ steht. Z bedeutet besonders Cl oder Br. Hierbei kann das Metallhalogenid in Substanz oder in Form einer Suspension in einem inerten Lösungsmittel zugegeben werden. Die Reaktionstemperatur beträgt vorzugsweise 0 bis -80°C.

Die in der Reaktionsstufe b) verwendeten elektrophilen Verbindungen sind bekannt oder können nach bekannten Verfahren hergestellt werden. Einige Beispiele sind Chlor- oder Bromcyan, Kohlendioxid, Carbonsäuredichlorid oder -bromid, Chlorkohlensäuremethylester, Bromkohlensäureethylester; Formylierungsreagenzien bevorzugt aus der Gruppe C₁-C₆-Alkoxymethylenaniline, Formylhalogenid, besonders -chlorid und -bromid, Ameisensäureester mit bevorzugt 1 bis 6, besonders 1 bis 4 C-Atomen im Alkoholrest, z.B. Ameisensäuremethyl- oder -ethylester, Orthoameisensäureester mit bevorzugt 1 bis 6 C-Atomen im Alkoholrest, N-disubstituierte Formamide mit 2 bis 12, bevorzugt 2 bis 6 C-Atomen und gegebenenfalls -O- oder N-C₁-C₄-Alkylgruppen im Amidrest, z.B. Dimethyl-, Diethyl-, Methylmethoxyformamid, Ameisensäuremorpholid oder Ameisensäure-N-methylpiperazid; Formaldehyd, Acetaldehyd, Benzaldehyd, Essigsäurechlorid oder -bromid, Propionsäurechlorid, Essigsäuremethylester, Propionsäureethylester, Dimethylacetamid, N-Methyl-N-methoxypropionsäureamid, Benzoesäureethylester, N,N-Dimethylphenylessigsäureamid, Chlor- oder Bromessigsäureethylester, 2-Chlor- oder Bromethanol, 2-Chlor- oder 2-Brompropan-1-ol, 4-Chlorbutan-1-ol, 3-Chlor- oder 3-Brompropinsäurenitril, 1-Chlor- oder 1-Brom-3,3,3-trifluorpropin, 3-Chlorpropincarbonsäureethylester, Ethoxyacetonitril, 1-Ethoxy-3,3,3-trifluorprop-1-en, Ethylenoxid, Propylenoxid, β-Dimethylaminoacrylsäureethylester, β-Tosyloxyacrylnitril, N,N-Dimethyl-β-chloracrylsäureamid, β-Aethoxy-acrylnitril, β-Tosyloxy-α-cyanoacrylnitril, N,N-Dimethylaminomethylencyanessigsäureethylester, α-Trifluormethyl-β-chloracrylnitril, Ethoxymethylencyanessigsäureethylester, Tosyloxymethylencyanessigsäureethylester, Δ3-1-Acetyl-3-cyano-4-chlor-pyrrolin, Δ3-1-Trimethylsilyl-3-cyano-4-tosyloxy-pyrrolin-2-on, 1-Acetyl-3-cyano-4-chlor-pyrrol, Trimethylborat, Triphenylborat, Trimethylchlorsilan, Diphenylmethylchlorsilan, Schwefel. Formylierungsreagenzien sind von Olah et. al. in Chem. Rev. 1987, 87, S. 671 beschrieben.

Es kann vorteilhaft sein, die Reaktion b) in Gegenwart von Pd-, Rh- oder Ru-Verbindungen durchzuführen.

Die Reaktionstemperatur gemäss Stufe b) kann z.B. -80°C bis 100°C, vorzugsweise -80°C bis 50°C betragen. Die Zugabe der elektrophilen Verbindung kann in Substanz oder Lösung erfolgen, wobei das gleiche oder ein anderes Lösungsmittel wie in Reaktionsstufe a) verwendet werden kann.

Die Isolierung der Verbindungen der Formel I erfolgt nach an sich bekannten Verfahren, indem man nach der Filtration des Reaktionsgemisches das Filtrat destilliert, kristallisiert oder chromatographiert. Es ist allgemein zweckmässig, das Reaktionsgemisch zu hydrolisieren, z.B. mit verdünnten Mineralsäuren, wie zum Beispiel, Salzsäure oder Schwefelsäure. Wenn die elektrophile Verbindung CO₂, ein Aldehyd, ein Epoxid, ein Carbonsäureamid oder -ester ist, werden zunächst Salze gebildet, die zur Isolierung der Verbindungen der Formel I eine Hydrolyse erforderlich machen.

Mit dem erfindungsgemässen Verfahren erhält man die Verbindungen der Formel I in hohen Ausbeuten und Reinheiten, wobei besonders keine oder nur ein unwesentliche Bildung von Stellungsisomeren beobachtet wird.

Die Verbindungen der Formeln I und Ia eignen sich zur Herstellung von Insektiziden bzw. Mikrobiziden. Die Carbonsäuren und Carbonsäurederivate, Aldehyde und Ketone können mittels üblicher reduktiver Methoden zu den entsprechenden Alkoholen reduziert werden. Aus den Alkoholen können mit Pyrethroid- oder pyrethroidähnlichen Carbonsäuren insektizide Ester hergestellt werden, wie sie in der DE-OS 2, 819, 788 (Beispiel 1) beschrieben sind.

Verbindungen der Formel I dienen weiterhin als Zwischenprodukte für wertvolle Herbizide der Sulfonylharnstoff-Klasse, wie sie beispielsweise in der EP-A-Nr. 99 339 beschrieben werden.

In Verbindungen mit Resten der Formel II kann die -CF₃- Gruppe gemäss dem in der US-A-4 705 801 beschriebenen Verfahren in die Nitrilgruppe umgewandelt werden. Die Umwandlung von Ester- und Amidgruppen in die Nitrilgruppe ist bekannt, ebenso die Hydrierung von Acetylenen zu Ethylenen. Durch Anwendung der bekannten Methoden können Verbindungen der Formel I mit der Gruppe R¹ gleich -CH=CR⁴R⁵ erhalten werden, worin R⁴ H, -CN oder -COOR² und R⁵ -CN oder -COOR² bedeuten. Diese Verbindungen sind Zwischenprodukte für die Mikrobiozide, deren Herstellung unter Verwendung dieser Zwischenprodukte in der EP-A-0 206 999 beschrieben ist.

Die nachfolgenden Beispiele erläutern die Erfindung näher.

### Beispiel 1: Herstellung von 4-(Trifluoracetyl)-2,2-difluor-1,3-benzodioxol.

In einem 250 ml 3-Halskolben werden unter Argon 7,9 g (50 mmol) 2,2-Difluor-1,3-benzodioxol in einem Gemisch von je 30 ml Tetrahydrofuran und Diethylether gelöst und bei -70°C tropfenweise während 20 Min. mit 40 ml (55 mmol) tert.-Butyllithium (1,39 M in Pentan) versetzt. Nach beendetem Zutropfen wird die gelbe Suspension 40 Min. bei -70°C gehalten. Danach wird innerhalb von 10 Min. eine Lösung von 9,8 g (50 mmol) α,α,α-Trifluoracetyl-N-methyl-piperazid in 20 ml Diethylether bei dieser Temperatur zugetropft. Anschliessend lässt man die trübe Lösung auf 0°C auftauen und hydrolysiert mit 30 ml 2N Salzsäure. Die wässrige Phase wird abgetrennt und 2mal mit je 60 ml Diethylether extrahiert. Die organischen Lösungen werden je einmal mit 50 ml 2N HCl und 50 ml H₂O gewaschen, über MgSO₄ getrocknet und im Vakuumrotationsverdampfer eingeengt. Bei der Destillation des Rückstands gehen beim Siedepunkt (Sdp.) 108-110°C/40 mbar 9,1 g (72 %) des Produktes als farblose Flüssigkeit über.
- ¹H-NMR (CDCl₃; 300 MHz):: 7,62 (d x d; J 7,9; 1,5; 1H); 7,29 (d x d; J 7,9; 1,5; 1H); 7,16 (t; J 7,9; 1H).

### Beispiel 2: Herstellung von 2,2-Difluor-1,3-benzodioxol-4-carbaldehyd.

a) In einem 500 ml 3-Halskolben werden unter Stickstoff 69 ml (110 mmol) n-Butyllithium (1,60 M in Hexan) vorgelegt und unter Eis/Kochsalz-Kühlung während 15 Min. mit einer Lösung von 13 g (110 mmol) N,N,N',N'-Tetramethylethylendiamin (TMEDA) in 30 ml Hexan versetzt. Zu der klaren, hellgelben Lösung werden innerhalb von 30 Min. 15,8 g (100 mmol) 2,2-Difluor-1,3-benzodioxol, gelöst in 120 ml Hexan, bei -10°C zugetropft, wobei sich allmählich 2,2-Difluor-1,3-benzodioxol-4-yllithium als voluminöser Niederschlag absetzt. Nach 30-minütigem kräftigem Rühren bei -10°C wird die weisse Suspension mit 20 ml (260 mmol) Dimethylformamid (DMF) versetzt. Das ölige Reaktionsgemisch lässt man 15 Min. bei -10°C rühren und hydrolisiert anschliessend mit 50 ml 10%-iger Salzsaure. Die wässerige Phase wird abgetrennt und 2mal mit je 100 ml Diethylether extrahiert. Die organischen Lösungen werden 3mal mit je 40 ml 1N HCl gewaschen, über MgSO₄ getrocknet und im Vakuumrotationsverdampfer bei 50°C/200 mbar eingeengt. Die Destillation liefert 16,8 g (90 %) farbloses Produkt vom Siedepunkt (Sdp.) 105-106°C/50 mbar.
b) Man verfährt wie in Beispiel 3 und verwendet Dimethylformamid anstelle von N-Methyl-N-methoxy-acetamid. Man erhält 2,2-Difluor-1,3-benzodioxol-4-carbaldehyd in einer Ausbeute von 73 %.
c) Zu 29,5 g (254 mmol) N,N,N',N'-Tetramethylethyldiamin und 40,0 g (253 mmol) 2,2-Difluor-1,3-benzodioxol in 35 ml Toluol werden unter Stickstoffatmosphäre bei -15° bis -10°C 94,0 g (272 mmol) n-Butyllithium (18,5 % in Tolulol) innert 1,5 Stunden zugetropft, wobei eine organge Suspension entsteht. Anschliessend werden 19,7 g (270 mmol) DMF bei - 15° bis -10°C innert 1/2 Stunde zudosiert. Die hellgelbe Suspension wird bei +10°C auf 346 g (1,105 mol) 11,6 proz. wässrige Salzsäure gegeben und 1/2 Stunde ausgerührt, die wässrige Phase abgetrennt und die organische Phase unter Stickstoff am Vakuumrotationsverdampfer bei 50°C/200 mbar eingedampft, was das erwünschte Produkt ergibt.
d) Zu 137,8 g (409 mmol) n-Butyllithiumlösung (19,0 % in Toluol) werden unter Stickstoffatmosphäre bei -15 bis -10°C 39,3 g (339 mmol) N,N,N',N'-Tetramethylethylendiamin zugetropft. Das entstandene Reaktionsgemisch wird innert 3 1/2 Stunden bei -15 bis -10°C unter Stickstoffatmosphäre zu einer Lösung von 53,3 g (337 mmol) 2,2-Difluor-1,3-benzodioxol in 46 ml Toluol zudosiert. Anschliessend werden 30 g (410 mmol) N,N-Dimethylformamid bei -15 bis -10°C innert 1/2 Stunde zugegeben. Die entstandene Lösung wird auf 461,1 g (1,472 mol) 11,6 proz. wässrige Salzsäure gegeben und 1/2 Stunde ausgerührt, die wässrige Phase abgetrennt und die organische Phase unter Stickstoff am Vakuumrotationsverdampfer bei 50°C/200 mbar eingedampft, was den erwünschten Aldehyd ergibt.

- ¹H-NMR (CDCl₃; 300 MHz):: 10,20 (s; 1H); 7,58 (d x d; J 7,9; 1,5; 1H); 7,34 (d x d; J 7,9; 1,5; 1H); 7,23 (t; J 7,9; 1H).

### Beispiel 3: Herstellung von 4-Acetyl-2,2-difluor-1,3-benzodioxol.

Zu einer in einem 250 ml 3-Halskolben vorliegenden Lösung von 8,0 g (50 mmol) 2,2-Difluor-1,3-benzodioxol in 10 ml Tetrahydrofuran (THF) wird unter Argon während 20 Min. bei -30°C 6,2 g (55 mmol), Kalium-tert.-butylat, gelöst in 40 ml THF, getropft. Danach wird das Gemisch auf -90°C (Methanol/flüssiger Stickstoff) abgekühlt und während 30 Min. mit 35 ml (55 mmol) n-Butyllithium (1,58 M in Hexan) versetzt. Die tiefrote 2,2-Difluor-1,3-benzodioxol-4-yl-kalium-Lösung wird 20 Min. bei -78°C gehalten. Anschliessend wird diese innerhalb von 15 Min. bei -78°C mit 5,2 g (50 mmol) N-Methyl-N-methoxy-acetamid in 20 ml THF versetzt. Nach beendetem Zutropfen lässt man das beige Reaktionsgemisch auf -10°C auftauen und hydrolisiert danach mit 40 ml 10%-iger Salzsäure. Das hydrolisierte Gemisch wird 3mal mit je 70 ml Diethylether extrahiert. Die organischen Lösungen werden 3mal mit je 30 ml 1N HCl gewaschen, über Na₂SO₄ getrocknet und im Vakuumrotationsverdampfer eingeengt. Bei der Destillation des Rückstandes gehen beim Siedepunkt (Sdp.) 114-116°C/30 mbar 7,1 g (79 %) des Produktes als farbloses Oel über.
- ¹H-NMR (CDCl₃; 300 MHz):: 7,65 (d x d; J 7,9; 1,5; 1H); 7,27 (d x d; J 7,9; 1,5; 1H); 7,18 (t; J 7,9; 1H); 2,68 (s; 3H).

### Beispiel 4: Herstellung von 2,2-Dimethyl-1-(2,2-difluor-1,3-benzodioxol-4-yl)-propan-1-ol.

a) Zu einer in einem 250 ml 3-Halskolben vorgelegten Lösung von 4,75 g (30 mmol) 2,2-Difluor-1,3-benzodioxol in 20 ml Tetrahydrofuran (THF) und 15 ml Diethylether werden unter Argon während 20 Min. 23,7 ml (33 mmol) tert.-Butyllithium (1,4 M in Pentan) bei -60°C zugetropft. Danach wird die gelbe, trübe Lösung 30 Min. bei -65°C gehalten und anschliessend innerhalb von 10 Min. mit einer feinen Suspension von 6,45 g (35 mmol) wasserfreiem Magnesiumbromid in 35 ml THF (hergestellt aus 1,2 Dibromethan und Magnesium in THF) versetzt. Die grünlich-blaue Lösung lässt man auf 0°C auftauen und tropft anschliessend 2,6 g (30 mmol) Pivalaldehyd, gelöst in 10 ml THF, zu. Nach 30 minütigem Rühren bei 25°C wird das farblose Reaktionsgemisch mit 30 ml 10%-iger Salzsäure hydrolisiert. Die Wasserphase wird abgetrennt und 2mal mit je 50 ml Diethylether extrahiert. Die organischen Lösungen werden je einmal mit 30 ml 2N HCl und 30 ml H₂O gewaschen, über MgSO₄ getrocknet und im Vakuumrotationsverdampfer eingeengt. Der kristalline, gelbe Rückstand wird aus Hexan umkristallisiert. Man erhält 5,1 g (70 %) farblose Plättchen vom Schmelzpunkt (Smp.) 68-69°C.
b) Man verfährt wie in Beispiel 1 und verwendet Pivalaldehyd anstelle von Trifluoracetyl-N-methylpiperazid. Man erhält 2,2-Dimethyl-1-(2,2-difluor-1,3-benzodioxol-4-yl)-propan-1-ol in einer Ausbeute von 81 %.

- ¹H-NMR (CDCl₃; 300 MHz):: 7,13 (d x d x d; J 8,0; 1,5; 0,5; 1H); 7,07 (t; J 8,0; 1H); 6,97 (d x d; J 8,0; 1,5; 1H); 4,63 (s; 1H); 2,05 (bs; OH); 0,95 (s; 9H).

### Beispiel 5: Herstellung von (E)-2-Cyano-3-(2,2-difluor-1,3-benzodioxol-4-yl)-2-propensäureethylester.

a) 7,9 g (50 mmol) 2,2-Difluor-1,3-benzodioxol werden gemäss Beispiel 2 mit 35 ml (55 mmol) n-Butyllithium (1,60 M in Hexan) und 6,5 g (55 mmol) TMEDA in 60 ml Hexan bei -15°C metalliert. Das ausgefallene 2,2-Difluor-1,3-benzodioxol-4-yllithium wird innerhalb von 30 Min. mit einer Lösung von 9,3 g (55 mmol) Ethoxymethylencyanessigsäureethylester in 30 ml Tetrahydrofuran bei -20°C versetzt, wobei sich bei der exotherm verlaufenden Reaktion eine orange-rote trübe Lösung bildet. Nach 20-minütigem Rühren bei -15°C wird mit 50 ml 2N Salzsäure hydrolysiert. Die Wasserphase wird 2mal mit je 80 ml Diethylether extrahiert. Die organischen Lösungen werden 2mal mit je 50 ml Wasser gewaschen, über MgSO₄ getrocknet und im Vakuumrotationsverdampfer zur Trockene eingedampft. Der Rückstand wird aus Ethanol/Wasser 4:1 kristallisiert. Es resultieren 10,1 g (72 %) farblose Plättchen vom Smp. 87-88°C.
b) 40,0 g (253 mmol) 2,2-Difluor-1,3-benzodioxol werden in 29,5 g (254 mmol) TMEDA und 35 ml Toluol mit 106,2 g (307 mmol) n-Butyllithiumlösung (18,5 % in Toluol) analog Beispiel 2c) bei -15 bis -10°C metalliert. Innert 2 Stunden werden 52,1 g (308 mmol) Ethoxymethylencyanessigsäureethylester in 115 ml Toluol bei -15 bis -10°C zum Reaktionsgemisch gegeben. Die entstandene Suspension wird nach 20 Minuten bei +10°C auf 350 ml Wasser gegeben, die wässrige Phase abgetrennt und die organische Phase am Vakuumrotationsverdampfer eingedampft, was das erwünschte Produkt ergibt.
c) 53,3 g (337 mmol) 2,2-Difluor-1,3-benzodioxol in 46 ml Toluol werden gemäss Beispiel 2d) mit 137,8 g (409 mmol) n-Butyllithiumlösung (19,0 % in Toluol) in Gegenwart von 39,3 g (339 mmol) TMEDA bei -15 bis -10°C metalliert. 69,1 g (409 mmol) Ethoxymethylencyanessigsäuremethylester in 150 ml Toluol werden bei -15 bis -10°C innert 2 1/2 Stunden zudosiert.

Das Reaktionsgemisch wird bei +10°C auf 450 ml Wasser gegeben, die Phasen getrennt und die organische Phase am Vakuumrotationsverdampfer eingedampft, wobei die Titelverbindung erhalten wird.
- ¹H-NMR (CDCl₃; 300 MHz):: 8,35 (s; 1H), 8,10 (m; X von ABX; 1H); 7,25 (m; AB von ABX; 2H); 4,42 (q; J 7,0; 2H); 1,42 (t; J; 7,0; 3H).

### Beispiel 6: Man verfährt wie in Beispiel 4 und verwendet β-Tosyloxyacrylnitril anstelle von Pivalaldehyd. Man erhält β-(2,2-Difluor-1,3-benzodioxol-4-yl)acrylnitril in einer Ausbeute von 52 %.

### Beispiel 7: Man verfährt wie in Beispiel 1 und leitet anstatt der Zugabe von Trifluoracetyl-N-methylpiperazid CO₂ ein. Man erhält 2,2-Difluor-1,3-benzodioxol-4-carbonsäure in einer Ausbeute von 61 %.

### Beispiel 8: Herstellung von 4-Hydroxy-2,2-difluor-1,3-benzodioxol.

15,8 g (100 mmol) 2,2-Difluor-1,3-benzodioxol werden gemäss Beispiel 2 mit 70 ml (110 mmol) n-Butyllithium (1,58 M in Hexan) und 12,8 g (110 mmol) TMEDA in 120 ml Hexan bei -20°C metalliert. Zu der weissen Suspension wird innerhalb von 5 Minuten eine Lösung von 10,4 g (100 mmol) Trimethylborat in 50 ml Diethylether bei -100°C zugetropft. Nach dem Auftauen und 30-minütigem Rühren bei Raumtemperatur wird das ausgefallene Dimethoxy-(2,2-difluor-1,3-benzodioxol-4-yl)boran unter Eiskühlung mit 35 ml 3 N Natronlauge und gleich anschliessend innerhalb von 10 Minuten mit 32 ml (∼310 mmol) 30%iger Wasserstoffperoxidlösung versetzt. Die gelb-orange Emulsion wird mit 10 %iger HCl auf pH 3 gestellt. Die wässrige Phase wird 3mal mit je 150 ml Diethylether extrahiert. Die organischen Lösungen werden 3mal mit je 100 ml 2N FeSO₄-Lösung, dann 2mal mit je 100 ml 20%iger Natriumbisulfitlösung und zuletzt mit 50 ml Sole gewaschen, über MgSO₄ getrocknet und im Vakuumrotationsverdampfer eingeengt. Bei der Destillation des Rückstandes gehen beim Siedepunkt (Sdp.) 135-139°C/25 mbar 8,4 g (48 %) des Produktes als hellgelbes Oel über.
- ¹H-NMR (CDCl₃; 300 MHz):: 6,93 (t; J 8,1; 1H); 6,67 (d x d; J 8,1; 1,0; 1H); 6,64 (d x d; J 8,1; 1,0; 1H); 6,15 (bs; OH).

### Beispiel 9: Herstellung von 2,2-Difluor-1,3-benzodioxol-4-boronsäure.

7,9 g (50 mmol) 2,2-Difluor-1,3-benzodioxol werden gemäss Beispiel 1 mit 40 ml tert.-Butyllithium (1,38 M in Pentan) in einem Gemisch von 55 ml Diethylether und 30 ml THF bei -70°C metalliert. Die Aryllithiumverbindung wird danach innerhalb von 2 Minuten mit 5,1 g (50 mmol) Trimethylborat in 25 ml Diethylether bei -100°C versetzt. Danach lässt man die gelbe, klare Reaktionslösung auf Raumtemperatur auftauen und hydrolysiert nach 30-minütigem Rühren unter Eiskühlung mit 100 ml 2N HCl. Die wässrige Phase wird 3 mal mit je 75 ml Diethylether extrahiert. Die organischen Lösungen werden mit Wasser und Sole neutral gewaschen und ohne zu trocknen im Vakuumrotationsverdampfer eingeengt. Der gelbe, kristalline Rückstand wird aus Petrolether umkristallisiert. Man erhält 7,4 g (73 %) hellgelbe Prismen vom Schmelzpunkt (Smp.) 103-104°C.
- ¹H-NMR (CDCl₃; 300 MHz):: 7,51 (m; X von ABX; 1H);
7,18 (m; AB von ABX; 2H);
5,33 (bs; 2 x OH).

### Beispiel 10: Herstellung von 2,2-Difluor-1,3-benzodioxol-4-thiol.

4,8 g (30 mmol) 2,2-Difluor-1,3-benzodioxol werden wie in Beispiel 1 mit 24 ml (33 mmol) tert.-Butyllithium (1,38 M in Pentan) in einem Gemisch von 35 ml THF und 15 ml Diethylether metalliert. Danach werden während 10 Minuten portionenweise 1,1 g (34 mmol) Schwefel (S₈) bei -30°C zugegeben. Anschliessend wird die orange Reaktionslösung noch 20 Minuten bei -30°C gehalten, dann mit 30 ml 2N HCl hydrolysiert. Die Wasserphase wird 2mal mit je 50 ml Diethylether extrahiert. Die organischen Phasen werden mit Wasser und Sole neutral gewaschen, über MgSO₄ getrocknet und im Vakuumrotationsverdampfer eingeengt. Die fraktionierte Destillation des Rückstandes liefert 3,4 g (60 %) farbloses Produkt vom Siedepunkt (Sdp.) 72-74°C/20 mbar.
- ¹H-NMR (CDCl₃; 60 MHz):: 6,85 (m; 3H); 3,49 (s, SH).

### Beispiel 11: Herstellung von 4-(Chlordifluoracetyl)-2,2-difluor-1,3-benzodioxol.

39,5 g (250 mmol) 2,2-Difluor-1,3-benzodioxol werden gemäss Beispiel 2 mit 172 ml (275 mmol) n-Butyllithium (1,60 M in Hexan) und 32 g (275 mmol) TMEDA in 300 ml Hexan bei -15°C metalliert. Das ausgefallene 2,2-Difluor-1,3-benzodioxol-4-yllithium wird innerhalb von 40 Minuten mit einer Lösung von 50,0 g (250 mmol) α-Chlor-α,α-difluoracetylmorpholid in 100 ml THF bei -15°C versetzt, wobei sich allmählich ein voluminöser, weisser Niederschlag bildet. Die Suspension wird 1h bei 0°C gerührt, dann mit 120 ml 10%iger HCl hydrolysiert. Die Wasserphase wird 2 mal mit je 250 ml Diethylether extrahiert. Die organischen Lösungen werden 2mal mit je 100 ml 1N HCl gewaschen, über MgSO₄ getrocknet und im Vakuumrotationsverdampfer bei 50°C eingeengt. Die fraktionierte Destillation liefert 41,3 g (61 %) hellgelbes Produkt vom Siedepunkt (Sdp.) 93-95°C/25 mbar.
- ¹H-NMR (CDCl₃; 300 MHz):: 7,75 (d x d; J 8,0; 1,0; 1H);
7,39 (d x d; J 8,0; 1,0; 1H);
7,27 (t; J 8,0).

### Beispiel 12: Man verfährt wie in Beispiel 1 und verwendet Trimethylchlorsilan anstelle von Trifluoracetyl-N-methylpiperazid. Man erhält 4-Trimethylsilyl-2,2-difluor-1,3-benzodioxol (Sdp. 80-81°C/160 mbar) in einer Ausbeute von 73 %

- ¹H-NMR (CDCl₃; 300 MHz):: 7,09 (m; X von ABX); 7,05 (m; AB von ABX; 2H); 0,33 (s; 9H).

## Patentansprüche

1. Verfahren zur Herstellung von Verbindungen der Formel I 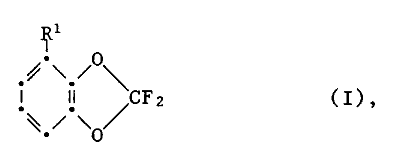 worin R¹ -OH, -SH, -CHO, -CN, -COOH, -B(OH)₂, -COX mit X gleich Cl oder Br, ferner -COOR², -SiR²₃ oder -B(OR²)₂ mit R² in der Bedeutung eines um die Hydroxylgruppe verminderten C₁-C₁₂-Alkoholrestes darstellt, worin R₁ ferner -CₙH₂ₙCOOR² mit n in der Bedeutung einer Zahl von 1 bis 4, oder lineares oder verzweigtes, unsubstituiertes oder mit -F, -CN, C₁-C₆-Alkoxy, Phenyl, Fluorphenyl, C₁-C₄-Alkoxy-phenyl, C₁-C₄-Alkylthio-phenyl, C₁-C₄-Alkyl-phenyl, C₁-C₄-Fluoralkyl-phenyl, Nitrophenyl, Cyanophenyl substituiertes C₁-C₁₂-Hydroxyalkyl bedeutet, oder worin R₁ einen unsubstituierten oder mit F, C₁-C₄-Alkoxy-, C₁-C₄-Alkylthio-, C₁-C₄-Alkyl-, C₁-C₄-Fluoralkyl-, Nitro, oder Cyano substituierten Benzylalkohol oder C₁-C₁₂-Acylrest darstellt, oder worin R₁ ein Rest der Formel II ist, 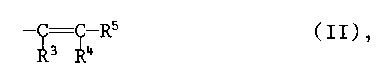 in welchem R⁵ für -CN, -CF₃, -COOR², -CONH₂, -CO-NHR² oder -CONR²₂ steht, R³ und R⁴ eine direkte Bindung oder je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 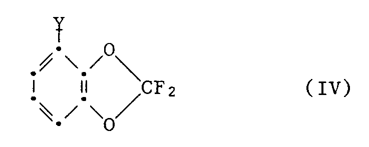 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄, BZ₃ oder ZrZ₄ zu einer Verbindung der Formel IV umsetzt, worin Y -Cu, -MgZ, -ZnZ, -CdZ, -CuZ, -PdZ, -NiZ, -AlZ₂, -SiZ₃, -SnZ, -SnZ₃, -TiZ₃, -BZ₂ oder -ZrZ₃ ist und Z -Cl, -Br oder -I und im Falle von Bor auch -OC₁-₄-Alkyl oder -O-Aryl bedeutet,
b) die Verbindung der Formel IV mit einer elektrophilen Verbindung aus der Gruppe X-CN, CO₂, S₈, COX₂, B(OR²)₃, X-SiR²₃, R²O-SiR²₃ oder XCOOR² mit X gleich -Cl oder -Br, einem Formylierungsreagens, CH₂O, einem Epoxid, einem unsubstituierten oder mit F, C₁-C₄-Alkoxy-, -Alkylthio, -Alkyl oder -Fluoralkyl, Nitro, Cyano substituierten Benzaldehyd, einem unsubstituierten oder mit -F, -Cl, -CN, C₁-C₆-Alkoxy, Phenyl, Fluorphenyl, C₁-C₄-Alkoxyphenyl, C₁-C₄-Alkylthiophenyl, C₁-C₄-Alkylphenyl, C₁-C₄-Fluoralkylphenyl, Nitrophenyl, Cyanophenyl substituierten C₁-C₁₁-Alkyl-CHO oder auch mit C₁-C₁₂-Acyl-X¹, worin X¹ -Cl, -Br, C₁-C₆-Alkoxy oder der Rest eines sekundären Amins ist, X²CₙH₂ₙCOOR² oder X²-CR³=CR⁴R⁵ worin X² eine Abgangsgruppe darstellt, umsetzt, und
c) die Verbindung der Formel I isoliert oder das Reaktionsgemisch zuerst hydrolisiert und dann die Verbindung der Formel I isoliert.

2. Verfahren zur Herstellung von Verbindungen der Formel I gemäss Anspruch 1, 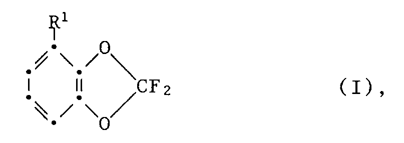 worin R¹ -CHO, -CN, -COOH, -COX mit X gleich Cl oder Br, -COOR² mit R² in der Bedeutung eines um die Hydroxylgruppe verminderten C₁-C₁₂-Alkoholrestes, -CₙH₂ₙCOOR² mit n in der Bedeutung einer Zahl von 1 bis 4, je lineares oder verzweigtes, unsubstituiertes oder mit -F, -CN, C₁-C₆-Alkoxy, Phenyl, Fluorphenyl, C₁-C₄-Alkoxyphenyl, C₁-C₄-Alkylthiophenyl, C₁-C₄-Alkylphenyl, C₁-C₄-Fluoralkylphenyl, Nitrophenyl, Cyanophenyl substituiertes C₁-C₁₂-Hydroxyalkyl oder C₁-C₁₂-Acyl, oder ein Rest der Formel II ist, 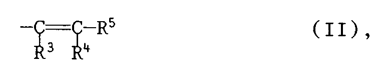 worin R⁵ für -CN, -CF₃, -COOR² oder -CONR²₂ steht, R³ und R⁴ je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 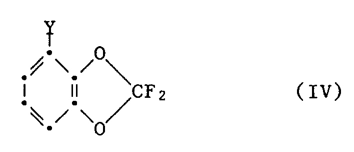 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄ oder ZrZ₄ zu einer Verbindung der Formel IV umsetzt, worin Y -Cu, -MgZ, -ZnZ, -CdZ, - CuZ, -PdZ, -NiZ, -AlZ₂, -SiZ₃, -SnZ, -SnZ₃, -TiZ₃ oder -ZrZ₃ ist und Z - Cl, -Br oder -I bedeutet,
b) die Verbindung der Formel IV mit einer elektrophilen Verbindung aus der Gruppe X-CN, CO₂, COX₂ oder XCOOR² mit X gleich -Cl oder -Br, einem Formylierungsreagens, CH₂O, einem unsubstituierten oder mit -F, - CN, C₁-C₆-Alkoxy, Phenyl, Fluorphenyl, C₁-C₄-Alkoxyphenyl, C₁-C₄-Alkyl-thiophenyl, C₁-C₄-Alkylphenyl, C₁-C₄-Fluoralkylphenyl, Nitrophenyl, Cyanophenyl substituierten C₁-C₁₁-Alkyl-CHO, einem Epoxid, einem unsubstituierten oder mit -F, C₁-C₄-Alkoxy-, -Alkylthio, -Alkyl oder -Fluoralkyl, Nitro, Cyano substituierten Benzaldehyd, C₁-C₁₂-Acyl-X¹, worin X¹ -Cl, -Br, C₁-C₆-Alkoxy oder der Rest eines sekundären Amins ist, X²CₙH₂ₙCOOR² oder X²-CR³=CR⁴R⁵ worin X² eine Abgangsgruppe darstellt, umsetzt, und
c) die Verbindung der Formel I isoliert oder das Reaktionsgemisch zuerst hydrolisiert und dann die Verbindung der Formel I isoliert.

3. Verfahren zur Herstellung von Verbindungen der Formel I gemäss Anspruch 2, worin R¹ -CHO, -CN, -COOH, -COX mit X gleich Cl oder Br, oder ein Rest der Formel II ist, 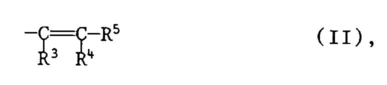 worin R⁵ für -CN, -CF₃, -COOR² oder -CONH₂ steht, R³ und R⁴ je H darstellen, oder R³ H ist und R⁴ unabhängig die Bedeutung von R⁵ hat, dadurch gekennzeichnet, dass man
a) in einem inerten Lösungsmittel 2,2-Difluorbenzo-1,3-dioxol mit einer Alkalimetallverbindung mit starker Anionbase zu einer Verbindung der Formel IV 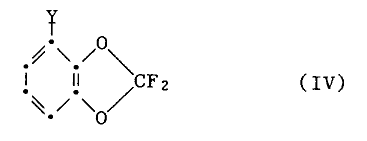 umsetzt, worin Y für ein Alkalimetall steht und gegebenenfalls darauf mit einem wasserfreien Metallhalogenid aus der Gruppe MgZ₂, ZnZ₂, CdZ₂, CuZ, CuZ₂, PdZ₂, NiZ₂, AlZ₃, SiZ₄, SnZ₂, SnZ₄, TiZ₄, BZ₃ oder ZrZ₄ zu einer Verbindung der Formel IV umsetzt, worin Y -Cu, -MgZ, -ZnZ, -CdZ, -CuZ, -PdZ, -NiZ, -AlZ₂, -SiZ₃, -SnZ, -SnZ₃, -TiZ₃, -BZ₂ oder -ZrZ₃ ist und Z -Cl, -Br oder -I bedeutet,
b) die Verbindung der Formel IV mit einer elektrophilen Verbindung aus der Gruppe X-CN, CO₂ oder COX₂ mit X gleich -Cl oder -Br, einem Formulierungsreagens oder X²-CR³=CR⁴R⁵, umsetzt, worin X² eine Abgangsgruppe darstellt, und
c) die Verbindung der Formel I isoliert oder das Reaktionsgemisch zuerst hydrolisiert und dann die Verbindung der Formel I isoliert.

4. Verfahren zur Herstellung von Verbindungen der Formel I gemäss Anspruch 3 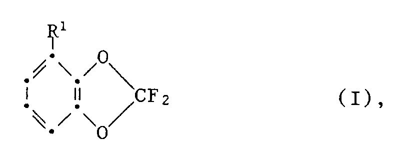 dadurch gekennzeichnet, dass R¹ -CHO, oder ein Rest der Formel II ist, 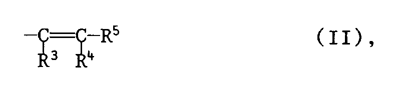 worin R⁵ für -CN, -COOR² oder -CONH₂ steht, R³ und R⁴ je H darstellen, oder R³ H ist und R⁴ unabhängig die Bedeutung von R⁵ hat.

5. Verfahren gemäss Anspruch 2, dadurch gekennzeichnet, dass die Umsetzung bei -150°C bis 150°C durchgeführt wird.

6. Verfahren gemäss Anspruch 2, dadurch gekennzeichnet, dass das Lösungsmittel ein unpolares oder polares aprotisches Lösungsmittel ist.

7. Verfahren gemäss Anspruch 6, dadurch gekennzeichnet, dass das Lösungsmittel ein aliphatischer oder aromatischer Kohlenwasserstoff, ein Ether, ein tertiäres Amin, ein N-alkyliertes Säureamid, ein Lactam oder ein cyclischer Harnstoff, ein Sulfoxid, ein Sulfon, ein Nitril oder Mischungen hiervon ist.

8. Verfahren gemäss Anspruch 2, dadurch gekennzeichnet, dass die Reaktionsmischung zusätzlich ein Komplexierungsmittel enthält.

9. Verfahren gemäss Anspruch 8, dadurch gekennzeichnet, dass das Komplexierungsmittel ein tertiäres Amin, ein N-substituiertes Carbonsäureamid, Lactam oder cyclischer Harnstoff, Ether oder ein Sulfoxid, ein Kronenether ein Alkalimetall-, Magnesium- oder Kupferhalogenid ist.

10. Verfahren gemäss Anspruch 2, dadurch gekennzeichnet, dass die Alkalimetallverbindung ein Li-, Na-, K-, Alkyl oder -Amid ist.

11. Verfahren gemäss Anspruch 2, dadurch gekennzeichnet, dass das Formylierungsreagens ein Formylhalogenid, ein Ameisensäureester, ein Orthoameisensäureester, ein Alkoxymethylenanilin oder ein N-disubstituiertes Formamid ist.

12. Verfahren gemäss Anspruch 2, dadurch gekennzeichnet, dass es sich bei X¹ als Rest eines sekundären Amins um 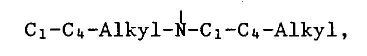 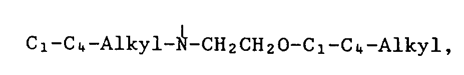 (C₁-C₄-AlkylOCH₂CH₂)₂N- oder den Rest eines 5- oder 6-gliedrigen heterocyclischen Amins, das ein weiteres Heteroatom aus der Gruppe -O-und =N-C₁-C₄-Alkyl enthalten kann, handelt.

13. Verfahren gemäss Anspruch 2, dadurch gekennzeichnet, dass X² als Abgangsgruppe ein Halogenid, C₁-C₆-Alkoxy, C₆-C₁₀-Aryloxy, C₁-C₁₂-Sekundäramino, C₁-C₈-Acyloxy oder der Rest einer Sulfonsäure ist.

14. Verfahren zur Herstellung von 2,2-Difluor-1,3-benzodioxol-4-carbaldehyd gemäss Anspruch 2, dadurch gekennzeichnet, dass man 2,2-Difluor-1,3-benzodioxol mit n-Butyllithium und anschliessend mit Dimethylformamid umsetzt.

## Claims

1. A process for the preparation of compounds of formula I 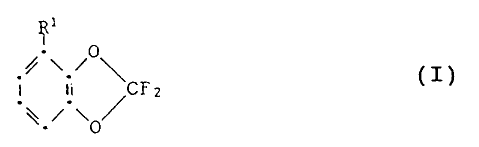 wherein R¹ is -OH, -SH, -CHO, -CN, -COOH, -B(OH)₂, -COX, with X being Cl or Br, or is -COOR², -SiR²₃ or -B(OR²)₂, with R² being a C₁-C₁₂alcohol moiety without the hydroxy group, wherein R¹ is further -CₙH₂ₙCOOR², with n being a number from 1 to 4, or linear or branched C₁-C₁₂hydroxyalkyl which is unsubstituted or is substituted by -F, -CN, C₁-C₆alkoxy, phenyl, fluorophenyl, C₁-C₄alkoxy-phenyl, C₁-C₄alkylthio-phenyl, C₁-C₄alkyl-phenyl, C₁-C₄fluoroalkyl-phenyl, nitrophenyl or by cyanophenyl, or wherein R¹ is a benzyl alcohol or a C₁-C₁₂acyl moiety, each unsubstituted or substituted by F, C₁-C₄alkoxy, C₁-C₄alkylthio, C₁-C₄-alkyl, C₁-C₄fluoroalkyl, nitro or by cyano, or wherein R¹ is a radical of formula II 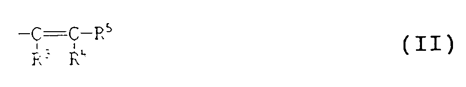 wherein R⁵ is -CN, -CF₃, -COOR², -CONH₂, -CO-NHR² or -CONR²₂, R³ and R⁴ are a direct bond or each is H, or R³ is H and R⁴ independently has the meanings of R⁵, which process comprises
a) in an inert solvent, reacting 2,2-difluorobenzo-1,3-dioxole with an alkali metal compound with a strong anion base to give a compound of formula IV 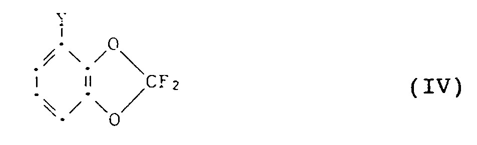 wherein Y is an alkali metal, and optionally then reacting with an anhydrous metal halide from the group MgZ₂, ZnZ₂, CdZ₂, CuZ, CuZ₂, PdZ₂, NiZ₂, AlZ₃, SiZ₄, SnZ₂, SnZ₄, TiZ₄, BZ₃ or ZrZ₄ to give a compound of formula IV wherein Y is -Cu, -MgZ, -ZnZ, -CdZ, -CuZ, -PdZ, -NiZ, -AlZ₂, -SiZ₃, -SnZ, -SnZ₃, -TiZ₃, -BZ₂ or -ZrZ₃ and Z is -Cl, -Br or -I and, in the case of boron, is also -OC₁-C₄alkyl or -O-aryl,
b) reacting the compound of formula IV with an electrophilic compound from the group X-CN, CO₂, S₈, COX₂, B(OR²)₃, X-SiR²₃, R²O-SiR²₃ or XCOOR², with X being -Cl or -Br, or with a formylating reagent, CH₂O, an epoxide, a benzaldehyde which is unsubstituted or is substituted by F, C₁-C₄alkoxy, C₁-C₄alkylthio, C₁-C₄alkyl, C₁-C₄fluoroalkyl, nitro or by cyano, a C₁-C₁₁alkyl-CHO which is unsubstituted or is substituted by -F, -Cl, -CN, C₁-C₆alkoxy, phenyl, fluorophenyl, C₁-C₄alkoxyphenyl, C₁-C₄alkylthiophenyl, C₁-C₄alkylphenyl, C₁-C₄fluoroalkylphenyl, nitrophenyl or by cyanophenyl, or with C₁-C₁₂acyl-X¹ wherein X¹ is -Cl, -Br, C₁-C₆alkoxy or the radical of a secondary amine, or with X²CₙH₂ₙCOOR² or X²-CR³=CR⁴R⁵ wherein X² is a leaving group, and
c) isolating the compound of formula I or first hydrolysing the reaction mixture and then isolating the compound of formula I.

2. A process for the preparation of compounds of formula I according to claim 1, 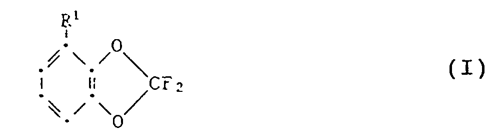 wherein R¹ is -CHO, -CN, -COOH, -COX, with X being Cl or Br, or is -COOR², with R² being a C₁-C₁₂alcohol moiety without the hydroxy group, or is -CₙH₂ₙCOOR², with n being a number from 1 to 4, or linear or branched C₁-C₁₂hydroxyalkyl or C₁-C₁₂acyl each unsubstituted or substituted by -F, -CN, C₁-C₆alkoxy, phenyl, fluorophenyl, C₁-C₄alkoxyphenyl, C₁-C₄alkylthiophenyl, C₁-C₄alkylphenyl, C₁-C₄-fluoroalkylphenyl, nitrophenyl or by cyanophenyl, or wherein R¹ is a radical of formula II 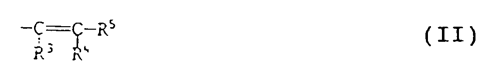 wherein R⁵ is -CN, -CF₃, -COOR² or -CONR²₂, R³ and R⁴ are each H, or R³ is H and R⁴ independently has the meanings of R⁵,
which process comprises
a) in an inert solvent, reacting 2,2-difluorobenzo-1,3-dioxole with an alkali metal compound with a strong anion base to give a compound of formula IV 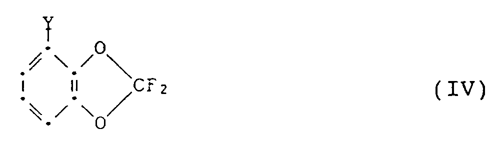 wherein Y is an alkali metal, and optionally then reacting with an anhydrous metal halide from the group MgZ₂, ZnZ₂, CdZ₂, CuZ, CuZ₂, PdZ₂, NiZ₂, AlZ₃, SiZ₄, SnZ₂, SnZ₄, TiZ₄ or ZrZ₄ to give a compound of formula IV wherein Y is -Cu, -MgZ, -ZnZ, -CdZ, -CuZ, -PdZ, -NiZ, -AlZ₂, -SiZ₃, -SnZ, -SnZ₃, -TiZ₃ or -ZrZ₃ and Z is -Cl, -Br or -I,
b) reacting the compound of formula IV with an electrophilic compound from the group X-CN, CO₂, COX₂ or XCOOR², with X being -Cl or -Br, or with a formylating reagent, CH₂O, a C₁-C₁₁alkyl-CHO which is unsubstituted or is substituted by -F, -CN, C₁-C₆alkoxy, phenyl, fluorophenyl, C₁-C₄alkoxyphenyl, C₁-C₄-alkylthiophenyl, C₁-C₄alkylphenyl, C₁-C₄fluoroalkylphenyl, nitrophenyl or by cyanophenyl, or with an epoxide, a benzaldehyde which is unsubstituted or is substituted by -F, C₁-C₄alkoxy, C₁-C₄alkylthio, C₁-C₄alkyl, C₁-C₄fluoroalkyl, nitro or by cyano, or with C₁-C₁₂acyl-X¹ wherein X¹ is -Cl, -Br, C₁-C₆alkoxy or the radical of a secondary amine, or with X²CₙH₂ₙCOOR² or X²-CR³=CR⁴R⁵ wherein X² is a leaving group, and
c) isolating the compound of formula I or first hydrolysing the reaction mixture and then isolating the compound of formula I.

3. A process for the preparation of compounds of formula I according to claim 2, wherein R¹ is -CHO, -CN, -COOH, -COX, with X being Cl or Br, or is a radical of formula II 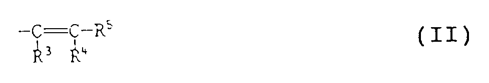 wherein R⁵ is -CN, -CF₃, -COOR² or -CONH₂, R³ and R⁴ are each H, or R³ is H and R⁴ independently has the meanings of R⁵,
which process comprises
a) in an inert solvent, reacting 2,2-difluorobenzo-1,3-dioxole with an alkali metal compound with a strong anion base to give a compound of formula IV 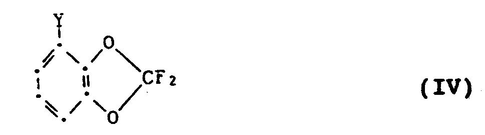 wherein Y is an alkali metal, and optionally then reacting with an anhydrous metal halide from the group MgZ₂, ZnZ₂, CdZ₂, CuZ, CuZ₂, PdZ₂, NiZ₂, AlZ₃, SiZ₄, SnZ₂, SnZ₄, TiZ₄, BZ₃ or ZrZ₄ to give a compound of formula IV wherein Y is -Cu, -MgZ, -ZnZ, -CdZ, -CuZ, -PdZ, -NiZ, -AlZ₂, -SiZ₃, -SnZ, -SnZ₃, -TiZ₃, BZ₂ or -ZrZ₃ and Z is -Cl, -Br or -I,
b) reacting the compound of formula IV with an electrophilic compound from the group X-CN, CO₂ or COX₂, with X being -Cl or -Br, or with a formylating reagent or with X²-CR³=CR⁴R⁵ wherein X² is a leaving group, and
c) isolating the compound of formula I or first hydrolysing the reaction mixture and then isolating the compound of formula I.

4. A process for the preparation of compounds of formula I according to claim 3, 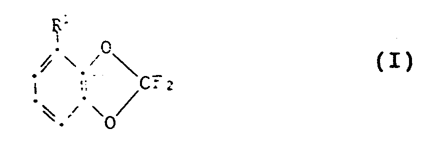 wherein R¹ is -CHO or a radical of formula II 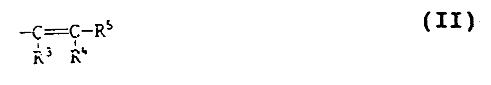 wherein R⁵ is -CN, -COOR² or -CONH₂, R³ and R⁴ are each H, or R³ is H and R⁴ independently has the meanings of R⁵.

5. A process according to claim 2, wherein the reaction is carried out at from -150°C to 150°C.

6. A process according to claim 2, wherein the solvent is a non-polar or polar aprotic solvent.

7. A process according to claim 6, wherein the solvent is an aliphatic or aromatic hydrocarbon, an ether, a tertiary amine, an N-alkylated acid amide, a lactam or a cyclic urea, a sulfoxide, a sulfone, a nitrile or a mixture thereof.

8. A process according to claim 2, wherein the reaction mixture contains, in addition, a complexing agent.

9. A process according to claim 8, wherein the complexing agent is a tertiary amine, an N-substituted carboxylic acid amide, a lactam or a cyclic urea, ether or a sulfoxide, a crown ether, an alkali metal halide, magnesium halide or copper halide.

10. A process according to claim 2, wherein the alkali metal compound is a Li, Na or K alkyl or amide.

11. A process according to claim 2, wherein the formylating reagent is a formyl halide, a formic acid ester, an orthoformic acid ester, an alkoxymethyleneaniline or an N-disubstituted formamide.

12. A process according to claim 2, wherein, as the radical of a secondary amine, X¹ is 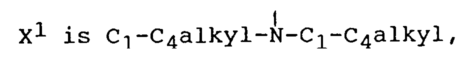 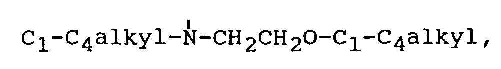 (C₁-C₄alkylOCH₂CH₂)₂N- or the radical of a 5- or 6-membered heterocyclic amine which may contain a further hetero atom from the group -O- and =N-C₁-C₄alkyl.

13. A process according to claim 2, wherein X² as a leaving group is a halide, C₁-C₆alkoxy, C₆-C₁₀aryloxy, C₁-C₁₂-secondary amino, C₁-C₈acyloxy or the radical of a sulfonic acid.

14. A process for the preparation of 2,2-difluoro-1,3-benzodioxole-4-carbaldehyde according to claim 2, which comprises reacting 2,2-difluoro-1,3-benzodioxole with n-butyllithium and then with dimethylformamide.

## Revendications

1. Procédé de préparation de composés de formule I 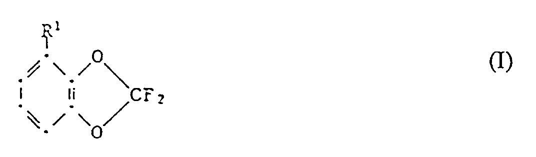 dans laquelle R¹ représente -OH, -SH, -CHO, -CN, -COOH, -B(OH)₂, -COX dans lequel X représente Cl ou Br, ou bien -COOR², -SiR²₃ ou -B(OR²)₂, R² représentant un radical d'alcool en C₁-C₁₂ d'où l'on a retiré le groupe hydroxy, R¹ pouvant en outre représenter -CₙH₂ₙCOOR², dans lequel n est un nombre allant de 1 à 4, ou un groupe hydroxyalkyle en C₁-C₁₂ à chaîne droite ou ramifiée, non substitué ou portant des substituants -F, -CN, alcoxy en C₁-C₆, phényle, fluorophényle, (alcoxy en C₁-C₄)-phényle, (alkylthio en C₁-C₄)-phényle, (alkyle en C₁-C₄)-phényle, (fluoralkyle en C₁-C₄)-phényle, nitrophényle, cyanophényle, ou bien R¹ représente un alcool benzylique ou un radical acyle en C₁-C₁₂ non substitué ou portant des substituants F, alcoxy en C₁-C₄, alkylthio en C₁-C₄, alkyle en C₁-C₄, fluoralkyle en C₁-C₄, nitro ou cyano, ou bien R¹ représente un groupe de formule II 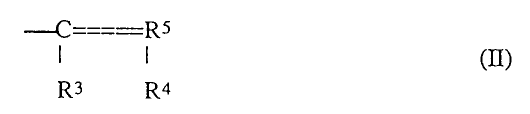 dans laquelle R⁵ représente -CN, -CF₃, -COOR², -CONH₂, -CO-NHR² ou -CONR²₂, R³ et R₄ représentent chacun une liaison directe ou H, ou bien R³ représente H et R⁴, indépendamment, a les significations de R⁵, caractérisé en ce que
a) on fait réagir dans un solvant inerte le 2,2-difluorobenzo-1,3-dioxole avec un dérivé de métal alcalin à anion fortement basique, ce qui donne un composé de formule IV 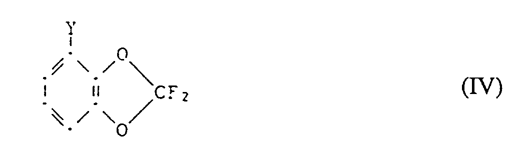 dans laquelle X représente un métal alcalin puis, le cas échéant, on fait réagir avec un halogénure métallique anhydre pris dans le groupe formé par MgZ₂, ZnZ₂, CdZ₂, CuZ, CuZ₂, PdZ₂,, NiZ₂,, AlZ₃, SiZ₄, SnZ₂, SnZ₄, TiZ₄ ou ZrZ₄, ce qui donne un composé de formule IV dans laquelle Y représente -Cu, -MgZ, -ZnZ, - CdZ, -CuZ, - PdZ, -NiZ, -AlZ_{2,}, -SiZ₃, -SnZ, -SnZ₃, TiZ₃ ou -ZrZ₃, et Z représente - Cl, -Br ou -I ou bien également, dans le cas du bore, -O-alkyle en C₁-C₄ ou -O-aryle,
b) on fait réagir le composé de formule IV avec un composé électrophile pris dans le groupe formé par X-CN, CO₂, S₈, COX₂, B(OR²)₃, X-SiR²₃, R²O-SiR²₃ ou XCOOR², X représentant -Cl ou -Br, un réactif formylant, CH₂O, un époxyde, un benzaldéhyde non substitué ou portant des substituants F, alcoxy en C₁-C₄, alkylthio en C₁-C₄, alkyle en C₁-C₄ ou fluoralkyle en C₁-C₄, nitro, cyano, un (alkyle en C₁-C₁₁)-CHO non substitué ou portant des substituants - F, -Cl, -CN, alcoxy en C₁-C₆, phényle, fluorophényle, (alcoxy en C₁-C₄)-phényle, (alkylthio en C₁-C₄)-phényle, (alkyle en C₁-C₄)-phényle, (fluoralkyle en C₁-C₄)-phényle, nitrophényle, cyanophényle, ou encore avec (acyle en C₁-C₁₂)-X¹, X¹ représentant -Cl, -Br, un groupe alcoxy en C₁-C₆ ou le radical d'une amine secondaire, X²CₙH₂ₙCOOR² ou X²-CR³=CR⁴R⁵, X² représentant un groupe éliminable, et
c) on isole le composé de formule I ou bien on hydrolyse d'abord le mélange de réaction puis on isole le composé de formule I.

2. Procédé de préparation des composés de formule I de la revendication 1 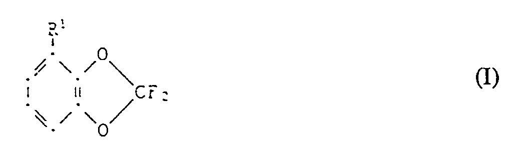 dans laquelle R¹ représente -CHO, -CN, -COOH, -COX, X représentant Cl ou Br, -COOR², R² représentant le radical d'un alcool en C₁-C₁₂ d'où l'on a retiré le groupe hydroxy, -CₙH₂ₙCOOR^{2,} n étant un nombre allant de 1 à 4, un groupe hydroxyalkyle en C₁-C₁₂ ou acyle en C₁-C₁₂ à chaîne droite ou ramifiée, non substitué ou portant des substituants -F, -CN, alcoxy en C₁-C₆, phényle, fluorophényle, (alcoxy en C₁-C₄)-phényle, (alkyle en C₁-C₄)-thiophényle, (alkyle en C₁-C₄)-phényle, (fluoralkyle en C₁-C₄)-phényle, nitrophényle, cyanophényle, ou bien un groupe de formule II 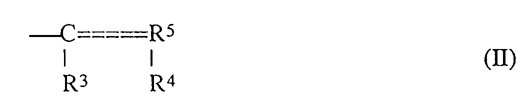 dans laquelle R⁵ représente -CN, -CF₃, -COOR² ou -CONR²₂, R³ et R⁴ représentent chacun H ou bien R³ représente H et R⁴, indépendamment, a les mêmes significations que R⁵, caractérisé en ce que
a) on fait réagir dans un solvant inerte le 2,2-difluorobenzo-1,3-dioxole avec un dérivé de métal alcalin à anion fortement basique, ce qui donne un composé de formule IV 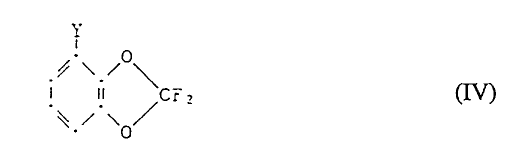 dans laquelle Y représente un métal alcalin puis, le cas échéant, on fait réagir avec un halogénure métallique anhydre pris dans le groupe formé par MgZ₂, ZnZ₂, CdZ₂, CuZ, CuZ₂, PdZ₂,, NiZ₂,, AlZ₃, SiZ₄, SnZ₂, SnZ₄, TiZ₄ ou ZrZ₄, ce qui donne un composé de formule IV dans laquelle Y représente -Cu, -MgZ, -ZnZ, - CdZ, -CuZ, - PdZ, -NiZ, -AlZ_{2,}, -SiZ₃, -SnZ, -SnZ₃, TiZ₃ ou -ZrZ₃, et Z représente - Cl, -Br ou -I,
b) on fait réagir le composé de formule IV avec un composé électrophile du groupe formé par X-CN, CO₂, COX₂ ou XCOOR², X représentant -Cl ou -Br, un réactif formylant, CH₂O, un (alkyle en C₁-C₁₁)-CHO non substitué ou portant des substituants -F, -CN, alcoxy en C₁-C₆, phényle, fluorophényle, (alcoxy en C₁-C₄)-phényle, (alkyle en C₁-C₄)-thiophényle, (alkyle en C₁-C₄)-phényle, (fluoralkyle en C₁-C₄)-phényle, nitrophényle, cyanophényle, un époxyde, un benzaldéhyde non substitué ou portant des substituants -F, alcoxy, alkylthio, alkyle ou fluoralkyle en C₁-C₄, nitro, cyano, (acyle en C₁-C₁₂)-X¹ dans lequel X¹ représente - Cl, -Br, un groupe alcoxy en C₁-C₆ ou le radical d'une amine secondaire, X²CₙH₂ₙCOOR² ou X²-CR³=CR⁴R⁵, X² représentant un groupe éliminable et
c) on isole le composé de formule I ou bien on hydrolyse d'abord le mélange de réaction puis on isole le composé de formule I.

3. Procédé de préparation des composés de formule I de la revendication 2, dans laquelle R¹ représente -CHO, -CN, -COOH, -COX, X représentant Cl ou Br, ou un groupe de formule II 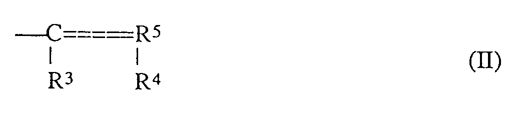 dans laquelle R⁵ représente -CN, -CF₃, -COOR² ou -CONH₂, R³ et R⁴ représentent chacun H ou bien R³ représente H et R^{4,} indépendamment, a les significations de R⁵, caractérisé en ce que
a) on fait réagir dans un solvant inerte le 2,2-difluorobenzo-1,3-dioxole avec un dérivé de métal alcalin à anion fortement basique, ce qui donne un composé de formule IV 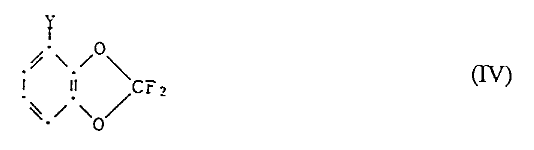 dans laquelle Y représente un métal alcalin puis, le cas échéant, on fait réagir avec un halogénure métallique anhydre pris dans le groupe formé par MgZ₂, ZnZ₂, CdZ₂, CuZ, CuZ₂, PdZ₂,, NiZ₂,, AlZ₃, SiZ₄, SnZ₂, SnZ₄, TiZ₄ ou ZrZ₄, ce qui donne un composé de formule IV dans laquelle Y représente -Cu, -MgZ, -ZnZ, - CdZ, -CuZ, - PdZ, -NiZ, -AlZ_{2,}, -SiZ₃, -SnZ, -SnZ₃, TiZ₃ ou -ZrZ₃, et Z représente - Cl, -Br ou -I,
b) on fait réagir le composé de formule IV avec un composé électrophile du groupe formé par X-CN, CO₂ ou COX₂, X représentant -Cl ou -Br, un réactif formylant ou X₂-CR³=CR⁴R⁵, X² représentant un groupe éliminable et
c) on isole le composé de formule I ou bien on hydrolyse d'abord le mélange de réaction puis on isole le composé de formule I.

4. Procédé de préparation des composés de formule I selon la revendication 3, 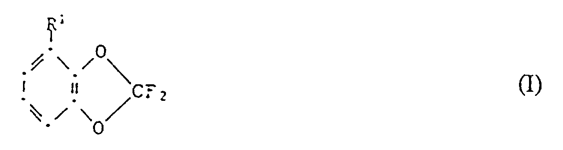 caractérisé en ce que R représente -CHO ou un groupe de formule II 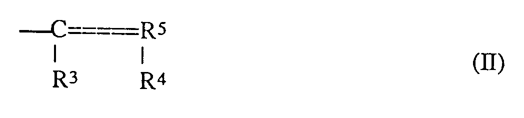 dans laquelle R⁵ représente -CN, -COOR² ou -CONH₂, R³ et R⁴ représentent chacun H ou bien R³ représente H et R⁴, indépendamment, a les significations de R⁵.

5. Procédé selon la revendication 2, caractérisé en ce que la réaction est effectuée entre -150 et +150°C.

6. Procédé selon la revendication 2, caractérisé en ce que le solvant est un solvant non polaire ou un solvant polaire aprotonique.

7. Procédé selon la revendication 6, caractérisé en ce que le solvant est un hydrocarbure aliphatique ou aromatique, un éther, une amine tertiaire, un N-alkylamide, un lactame ou une urée cyclique, un sulfoxyde, une sulfone, un nitrile ou leurs mélanges.

8. Procédé selon la revendication 2, caractérisé en ce que le mélange de réaction contient en outre un agent complexant.

9. Procédé selon la revendication 8, caractérisé en ce que l'agent complexant est une amine tertiaire, un carboxamide substitué à l'azote, un lactame ou une urée cyclique, un éther ou un sulfoxyde, un éther en couronne, un halogénure de métal alcalin, de magnésium ou de cuivre.

10. Procédé selon la revendication 2, caractérisé en ce que le dérivé de métal alcalin est un alkyl-Li, -Na, -K ou un amidure de Li, de Na ou de K.

11. Procédé selon la revendication 2, caractérisé en ce que le réactif formylant est un halogénure de formyle, un ester formique, un ester orthoformique, une alcoxyméthylène-aniline ou un formamide disubstitué à l'azote.

12. Procédé selon la revendication 2, caractérisé en ce que X¹ consiste en un radical d'amine secondaire de structure (alkyle en C₁-C₄)-N-alkyle en C₁-C₄, (alkyle en C₁-C₄)-N-CH₂CH₂O-alkyle en C₁-C₄, (alkyle en C₁-C₄-0 CH₂CH₂)₂N- ou en le radical d'une amine hétérocyclique à 5 ou 6 chaînons qui peut contenir un autre hétéroatome choisi parmi -O- et =N-alkyle en C₁-C₄.

13. Procédé selon la revendication 2, caractérisé en ce que X², substituant éliminable, est un substituant halogénure, alcoxy en C₁-C₆, aryloxy en C₆-C₁₀, sec.-amino en C₁-C₁₂, acyloxy en C₁-C₈ ou le radical d'un acide sulfonique.

14. Procédé de préparation du 2,2-difluoro-1,3-benzodioxole-4-carbaldéhyde selon la revendication 2, caractérisé en ce que l'on fait réagir le 2,2-difluoro-1,3-benzodioxole avec le n-butyllithium puis avec le diméthylformamide.
